# EUROPÄISCHE PATENTANMELDUNG

(11) **EP 2 630 973 A1**
(43) Veröffentlichungstag der Anmeldung: **28.08.2013**
(21) Anmeldenummer: 12156678.0
(22) Anmeldetag: 23.02.2012
(51) Int. Cl.: A61L 2/20

(54) **Verfahren und Vorrichtung zur Gassterilisation von Erzeugnissen bei dem die Prozessgase vor Einleiten in die Sterilisationskammer durch einen Mischer ohne bewegliche Teile homogenisiert werden**

(71) Anmelder: B. Braun Melsungen AG, 34212 Melsungen (DE)
(72) Erfinder: Geiger, Ralph, 34587 Felsberg (DE); Fischer, Franz, 34117 Kassel (DE)
(74) Vertreter: Arth, Hans-Lothar

(57) **Zusammenfassung**

Die vorliegende Erfindung beschreibt ein Verfahren sowie eine Vorrichtung zur Gassterilisation, bei dem Erzeugnisse mit einem Sterilisationsgas sterilisiert werden. Hierzu werden die Prozessgase vor dem Einleiten in die Sterilisationskammer in einem Mischer ohne bewegliche Teile vermischt, um ein homogenes Gasgemisch zu erhalten.

## Beschreibung

Die vorliegende Erfindung beschreibt ein Verfahren sowie eine Vorrichtung zur Gassterilisation, bei dem Erzeugnisse mit einem Sterilisationsgas sterilisiert werden. Hierzu werden die Prozessgase vor dem Einleiten in die Sterilisationskammer in einem Mischer ohne bewegliche Teile vermischt, um ein homogenes Gasgemisch zu erhalten.

Die Sterilisation mit Gasen ist ein häufig angewandtes und bekanntes Verfahren. Besonders weit verbreitet ist die Anwendung von Ethylenoxidgas, welches Bakterien, Schimmel und Pilze abtötet und daher gut geeignet ist für die Sterilisation von thermolabilen Substanzen. Aufgrund seiner explosiven und hochentzündlichen Eigenschaften wird Ethylenoxid (EO) häufig mit inert Gasen gemischt, wie z.B. mit Kohlendioxid, Stickstoff oder mit Halogenkohlenwasserstoffen. Dies kann sowohl beim Abfüllen des Gases in Flaschen (z.B. ein Gemisch aus 6 % EO / 94 % CO₂) als auch in der Sterilisationskammer erfolgen.

Das übliche Verfahren aus dem Stand der Technik sieht vor, dass sowohl das Ethylenoxid als auch das inert Gas in eine Sterilisationskammer geleitet werden und dort durch Verwirbelungen, die beim Einleiten der Prozessgase entstehen, miteinander vermischt werden. Diese Vermischung wird meist noch durch einen Ventilator unterstützt, der während des gesamten Sterilisationszyklus für eine Umwälzung des Gasgemischs sorgt. Vor Entnahme des Sterilisationsgutes aus der Kammer wird zum Schutze der Mitarbeiter (Explosions- und auch Gesundheitsschutz) das Ethylenoxid entsorgt und das Sterilisationsgut üblicherweise durch mehrfaches Spülen mit inert Gas von eingelagertem (eindiffundierten) Ethylenoxid befreit.

Die Homogenisierung der Prozessgase ist aufgrund des niedrigen Drucks und der damit verbundenen niedrigen Dichte in der Sterilisationskammer gering und erfolgt größtenteils nur außerhalb der Erzeugnisse. Dies kann dazu führen, dass die Prozessgase nicht ordentlich gemischt werden und es zu einer ungleichmäßigen Verteilung des Ethylenoxids in der Sterilisationskammer kommt. Im ungünstigsten Fall sammelt sich das Ethylenoxid aufgrund seiner höheren Dichte am Boden der Sterilisationskammer, was dazu führt, dass die notwendigen Konzentrationen für eine effektive Sterilisation in einzelnen Regionen - speziell im oberen Bereich der Sterilisationskammer - nicht mehr erreicht werden. Dies ist besonders kritisch bei medizinischen und medizintechnischen Produkten, bei der eine absolute Keimfreiheit unabdingbar ist und führt oft dazu, dass verlängerte Einwirkzeiten eingehalten werden müssen, um die Keimfreiheit der Erzeugnisse zu garantieren.

Der Einsatz von Ventilatoren, die genau dies verhindern sollen, ist technisch sehr aufwändig und teuer, da die Ventilatoren den Anforderungen des Explosionsschutzes genügen müssen. Alternativ wurde vorgeschlagen, die Prozessgase in einem extra Tank vor Zugabe in die Sterilisationskammer zu mischen. Auch dieser Ansatz ist mit erheblichen Kosten und apparativen Mehraufwand verbunden und kann nur unter Schwierigkeiten in bestehende Anlagen integriert werden.

Auch bei Sterilisationen mit anderen Gasen kann es zu Problemen aufgrund von inhomogener Verteilung des Gases kommen. Insbesondere die Temperaturverteilung ist z.B. bei der Dampfsterilisation ein entscheidender Prozessparameter. Bei dieser Sterilisationsart sind unter anderem zwei Phänomene bekannt, welche zu einer Prozessstörung führen können. Zum einen bilden inerte Gase, welche während der Dampfeingabe durch den Dampf mit eingegeben werden, partielle Gasblasen, welche dann die Dampfkondensation und somit Energiezuführung am Sterilisiergut stören. Zum anderen kann es durch hygroskopische Kondensation zu einer partiellen Überhitzung von stark getrockneten Materialien kommen, insbesondere von Textilien.

Es besteht daher ein Bedarf den Prozess der Gassterilisation zu verbessern, um eine homogenere Verteilung des Gasgemisches in der Sterilisationskammer zu erreichen.

Der Erfindung liegt somit die Aufgabe zugrunde, ein Verfahren zur Gassterilisation von Erzeugnissen sowie eine Vorrichtung bereitzustellen, durch die ein homogeneres Gasgemisch zur Gassterilisation erzeugt werden kann.

Es wurde überraschend gefunden, dass diese Aufgabe gelöst wird, indem zumindest ein Teil der Gase beim Einleiten in die Sterilisationskammer durch einen Mischer ohne bewegliche Teile geleitet werden. Weitere vorteilhafte Ausgestaltungen der Erfindung sind in den abhängigen Patentansprüchen, der Beschreibung, den Figuren sowie den Beispielen angegeben.

Die erfindungsgemäße Vorrichtung zur Gassterilisation von Erzeugnissen umfasst eine Sterilisationskammer und einen Mischer ohne bewegliche Teile.

In einer bevorzugten Ausführungsform hat die Sterilisationskammer mindestens eine Einlassöffnung und mindestens eine Auslassöffnung für Gase.

In einer weiteren Ausführungsform umfasst der Mischer ohne bewegliche Teile weiterhin einen Einlass, für ein oder mehrere Prozessgase, einen Auslass, zur Eingabe der Gase in die Sterilisationskammer und einen Einlass für Prozessgase, die aus der Sterilisationskammer kommen.

Bevorzugter Weise umfasst die erfindungsgemäße Vorrichtung zur Gassterilisation von Erzeugnissen eine Sterilisationskammer, die mindestens eine Einlassöffnung und mindestens eine Auslassöffnung für Gase aufweist, und einen Mischer ohne bewegliche Teile, wobei der Mischer ohne bewegliche Teile derart angeordnet ist, dass zumindest ein Teil der eingeleiteten Gase zuerst den Mischer ohne bewegliche Teile durchfließt, um anschließend über die mindestens eine Einlassöffnung in die Sterilisationskammer geleitet zu werden, wobei gleichzeitig zumindest ein Teil der Gase, welche sich bereits in der Sterilisationskammer befinden, über die mindestens eine Auslassöffnung aus der Sterilisationskammer dem Mischer ohne bewegliche Teile zugeleitet werden und sich dort mit einem oder mehreren der eingeleiteten Gase vermischt.

In einer weiteren Ausführungsform umfasst der Mischer ohne bewegliche Teile einen Einlass, für ein oder mehrere Prozessgase und einen Auslass, zur Eingabe der Gase in die Sterilisationskammer. In dieser Ausführungsform umfasst die erfindungsgemäße Vorrichtung zur Gassterilisation von Erzeugnissen eine Sterilisationskammer, die mindestens eine Einlassöffnung und mindestens eine Auslassöffnung für Gase aufweist, und einen Mischer ohne bewegliche Teile, wobei der Mischer ohne bewegliche Teile derart angeordnet ist, dass zumindest ein Teil der eingeleiteten Gase zuerst den Mischer ohne bewegliche Teile durchfließt, um anschließend über die mindestens eine Einlassöffnung in die Sterilisationskammer geleitet zu werden, wobei gleichzeitig zumindest ein Teil der Gase, welche sich bereits in der Sterilisationskammer befinden, über die mindestens eine Auslassöffnung aus der Sterilisationskammer in eine dem Mischer ohne bewegliche Teile gaszuleitende Rohrleitung zugeleitet werden, um sich dann mit dem oder den eingeleiteten Gas(en) im Mischer ohne bewegliche Teile zu vermischen.

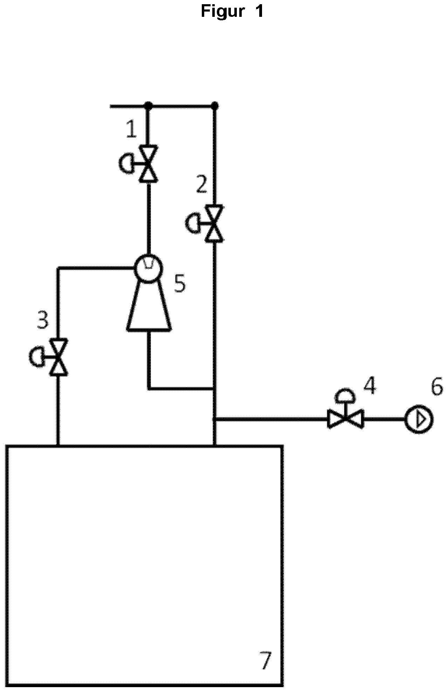

Mit Bezug auf die Figur 1 umfasst die Vorrichtung zur Gassterilisation bevorzugterweise einen Mischer ohne bewegliche Teile (5), Ventile (1, 3) und eine Sterilisationskammer (7), wobei die Sterilisationskammer (7) mindestens eine Einlassöffnung und mindestens eine Auslassöffnung aufweist und der Mischer ohne bewegliche Teile derart angeordnet ist, dass er über eine erste Leitung, die in einer ersten Öffnung im Mischer ohne bewegliche Teile mündet, mit der mindestens einen Auslassöffnung der Sterilisationskammer (7) verbunden ist und mit einer zweiten Leitung, die in einer zweiten Öffnung im Mischer ohne bewegliche Teile mündet, mit der mindestens einen Einlassöffnung der Sterilisationskammer (7) verbunden ist und eine dritte gaszuführende Leitung in eine dritte Öffnung im Mischer ohne bewegliche Teile mündet, wobei das Ventil (3) in der ersten Leitung zwischen der Auslassöffnung der Sterilisationskammer (7) und dem Mischer ohne bewegliche Teile (5) und das Ventil (1) in der dritten gaszuführenden Leitung zum Mischer ohne bewegliche Teile (5) angeordnet ist.

Bevorzugt umfasst die Vorrichtung zur Gassterilisation zusätzlich eine gaszuführende Bypass-Leitung mit einem Ventil (2), wobei die gaszuführende Bypass-Leitung derart angeordnet ist, dass sie den Mischer ohne bewegliche Teile überbrückt und direkt in der Sterilisationskammer (7) mündet.

Traditionell ist die Gassterilisation auch unter dem Begriff "Chemische Sterilisation" bekannt, wobei man sich hier die Wirkung von toxischen, flüchtigen Substanzen oder Gasen zu Nutze macht. Hierunter fallen bestimmte chemische Stoffe, wie z.B. Formaldehyd, Ethylenoxid, Peressigsäure oder Wasserstoffperoxid. Für die vorliegende Erfindung ist prinzipiell jede Substanz für die Gassterilisation geeignet, die sich im gasförmigen Zustand befindet bzw. sich in den gasförmigen Zustand überführen lässt und in diesem Zustand eine abtötende Wirkung auf Bakterien, Schimmel und/oder Pilze hat. Hierunter fällt insbesondere auch die Sterilisation mit Wasserdampf. Bevorzugterweise lässt sich solch eine Substanz nach erfolgter Sterilisation wieder leicht entfernen bzw. verflüchtigt sich selbständig.

Die Sterilisationskammer kann jegliche Ausmaßnahme annehmen und lässt sich bevorzugterweise dicht verschließen. In der Sterilisationskammer können unterschiedliche Parameter reguliert werden, wie z.B. der Druck und/oder die Temperatur. In der Sterilisationskammer wird das Sterilisiergut platziert und die Sterilisationskammer derart verschlossen, dass keine Gase unkontrolliert aus der Kammer entweichen können. Bevorzugterweise sollte das Sterilisiergut sauber und trocken sein und darüber hinaus vorzugsweise in speziell gasdurchlässige Folien verpackt werden. Die Gassterilisation mit chemischen Substanzen wird in der Regel bei thermolabilen Materialien eingesetzt, wobei thermostabile Materialien bevorzugterweise über eine Dampfsterilisation sterilisiert werden.

Die Sterilisationskammer weist mindestens eine Einlassöffnung und eine Auslassöffnung für Gase auf, wobei bevorzugterweise mehrere Einlassöffnungen und Auslassöffnungen vorhanden sein können. Je nach Größe und Form der Sterilisationskammer, angestrebter Sterilisationsdauer, der Form der Erzeugnisse und/oder dem verwendeten Sterilisationsgas können eine Vielzahl von Einlass- und Auslassöffnungen vorhanden sein. Bevorzugterweise befinden sich die Einlassöffnungen diagonal zu den Auslassöffnungen, damit kein Bypass in der Kammer entsteht.

In einer weiteren bevorzugten Ausführungsform wird das Gas nicht einfach frei in die Sterilisationskammer eingeleitet, sondern über ein oder mehrere Prallbleche, welche innerhalb der Sterilisationskammer unbeweglich angebracht sind, in der Sterilisationskammer verteilt.

Erfindungsgemäß wird zumindest ein Teil der Gase bzw. das Sterilisationsgas durch einen Mischer ohne bewegliche Teile in die Sterilisationskammer eingeleitet. Mischer ohne bewegliche Teile sind dadurch gekennzeichnet, dass im Inneren des Mischers keine beweglichen Elemente für die Mischung vorhanden sind. Konkret bedeutet dies, dass das Mischen ganz ohne bewegte oder bewegliche Teile auskommt und nur die sich zu mischenden Komponenten bewegt oder verwirbelt werden. Dies schließt natürlich nicht aus, dass generell bewegliche Teile an dem Mischer vorhanden sind, diese beweglichen Teile sind aber nicht an der Durchmischung der Gase beteiligt.

Wie hierin verwendet bedeutet "Mischer ohne bewegliche Teile", dass die mit dem Gas in Kontakt kommenden inneren Teile des Mischers nicht beweglich sind. Anders ausgedrückt, der "Mischer ohne bewegliche Teile" hat keine Teile, die durch Bewegung eine Durchmischung und/oder Förderung des Gases bewirken.

In einer bevorzugten Ausführungsform handelt es sich bei dem Mischer ohne bewegliche Teile um eine Strahlpumpe. Bevorzugterweise umfasst die Strahlpumpe eine Treibdüse, eine Mischkammer und einen Diffusor. In einer weiteren bevorzugten Ausführungsform umfasst die Strahlpumpe eine Treibdüse, eine Mischkammer, einen Diffusor, weiterhin noch einen Einlass, für ein oder mehrere Prozessgase, einen Auslass, zur Eingabe der Gase in die Sterilisationskammer und einen Einlass für Prozessgase, die aus der Sterilisationskammer kommen.

In einer weiteren bevorzugten Ausführungsform handelt es sich bei dem Mischer ohne bewegliche Teile um einen statischen Mischer. Statische Mischer sind dadurch gekennzeichnet, dass innerhalb des Mischers Leitelemente derart angeordnet sind, dass das Gas bzw. die Gase beim Durchströmen des Mischers innerhalb einer kurzen Fließstrecke durchmischt werden. Die Leitelemente bleiben dabei in fester Position, d.h. sind nicht beweglich und bewirken, dass die Komponenten über den Strömungsquerschnitt durchmischt werden.

Erfindungsgemäß ist es auch möglich nicht nur einen, sondern auch mehrere Mischer ohne bewegliche Teile einzusetzen. Dies ist unter anderem von Prozesseingabeparametern (z.B. Druck der Gase, Fließgeschwindigkeiten) abhängig. In einer bevorzugten Ausführungsform werden zwei Mischer ohne bewegliche Teile eingesetzt, bevorzugterweise drei Mischer ohne bewegliche Teile, weiterhin bevorzugt vier Mischer ohne bewegliche Teile und am meisten bevorzugt fünf Mischer ohne bewegliche Teile. Hierbei sind Kombinationen von unterschiedlichen Mischern ohne bewegliche Teile denkbar. Zum Beispiel kann ein statischer Mischer und eine Strahlpumpe gleichzeitig verwendet werden. Weiterhin möglich ist auch der Einsatz von Mischern ohne bewegliche Teile, die aus einer Kombination eines statischen Mischers und einer Strahlpumpe bestehen. In solch einem Fall wäre die Strahlpumpe weiterhin dadurch gekennzeichnet, dass sie Leitelemente aufweist, analog zu den Leitelementen eines statischen Mischers.

Durch den Einsatz eines Mischers ohne bewegliche Teile in einer Vorrichtung für die Gassterilisation wird der Sterilisationsprozess kürzer, günstiger und effektiver. Weiterhin wird erfindungsgemäß kein Ventilator benötigt, mit dem die Gase in der Sterilisationskammer oder auch während der Einleitung in die Sterilisationskammer miteinander verwirbelt werden. Erfindungsgemäß weisen die Mischer ohne bewegliche Teile daher zur Gasdurchmischung keine Bauteile auf, welche aufgrund Bewegung dieser Bauteile des Mischers wie z.B. Rotation die Gasdurchmischung bewirken.

Nachfolgend wird die Erfindung anhand der Zeichnungen erläutert. Es zeigen: Fig. 1 ein Fließschema mit einem Mischer ohne bewegliche Teile im Rohrleitungssystem einer Gassterilisationsvorrichtung, Fig.2 die schematische Ansicht eines Mischers ohne bewegliche Teile in Form einer Strahlpumpe, die Fig.3 eine schematische Darstellung für einen Anschluss einer Strahlpumpe an eine Sterilisationskammer, die Fig. 4 den Verlauf der Druckkurven während eines Sterilisationsprozesses.

In Fig. 1 ist ein Mischer ohne bewegliche Teile im Rohrleitungssystem einer Gassterilisationsvorrichtung abgebildet. Die Gassterilisationsvorrichtung besteht aus oder enthält einen Mischer ohne bewegliche Teile 5, eine Sterilisationskammer 7, ein Einlassventil 1, ein Einlassventil 2, ein Auslassventil 4 sowie eine Vakuumpumpe 6 und ein Mischventil 3. Die Eingabe der Prozessgase Ethylenoxid, Stickstoff und Luft erfolgt bevorzugterweise durch das Einlassventil 1 über den Mischer ohne bewegliche Teile 5. Alternativ können die Prozessgase auch am Mischer ohne bewegliche Teile 5 vorbei über das Ventil 2 eingeleitet werden (Bypass-Leitung). Die Luft wird aus der Umgebung angesaugt, kann aber auch über eine Druckluftleitung zugeführt werden. Die Lufteingabe erfolgt bevorzugterweise über die Ventile 1 und 2. Das Ethylenoxid liegt zuerst flüssig vor und wird über einen Wärmetauscher bzw. Verdampfer verdampft (nicht gezeigt). Die Ethylenoxid Eingabe erfolgt dann bevorzugterweise sowohl über das Ventil 1 als auch das Ventil 2, um die Prozesszeiten zu verkürzen, wobei die Eingabe aber auch nur über das Ventil 1 erfolgen kann. Die Homogenisierung des Gasgemisches vor der Ethylenoxid Einwirkzeit erfolgt bevorzugterweise durch die nachfolgende Stickstoffeinspeisung. Die erste Stickstoffeingabe vor der Dampfeingabe erfolgt bevorzugterweise über beide Ventile 1 und 2 (Bypass-Leitung und Mischer). Die zweite Stickstoffeingabe direkt nach der Ethylenoxid Einspeisung erfolgt nur über den Mischer ohne bewegliche Teile 5. Die nachfolgenden Stickstoffspülungen können über beide Ventile 1 und 2 erfolgen (Bypass-Leitung und Mischer). Die Dampfeinspeisung erfolgt bevorzugterweise durch das Einlassventil 1 über den Mischer ohne bewegliche Teile 5. Vorzugsweise wird der Mischer ohne bewegliche Teile 5 hauptsächlich für die Dampfeinspeisung und die Stickstoffeinspeisung nach der Ethylenoxid Eingabe genutzt.

Es ist für den Fachmann ersichtlich, dass das Rohrleitungssystem und die Ventile je nach Aufbau des Systems unterschiedlich angeordnet sein können. Zum Beispiel ist es möglich, dass die Rohrleitung mit dem Ventil 4 direkt von der Sterilisationskammer abgeht und nicht von der Ausgangsleitung des Mischers ohne bewegliche Teile. Diese und andere Variationen liegen im Können des durchschnittlichen Fachmanns und begründen keine erfinderische Tätigkeit.

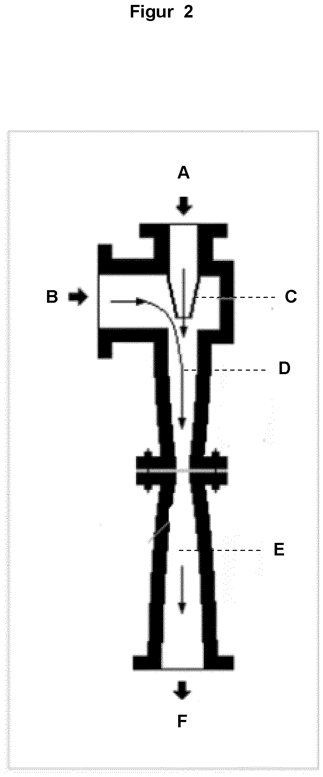

In Fig. 2 ist schematisch ein Mischer ohne bewegliche Teile in Form einer Strahlpumpe abgebildet, wie er in der erfindungsgemäßen Vorrichtung zum Einsatz kommen kann. Der Mischer ohne bewegliche Teile besteht in dieser Ausführungsform aus einer Treibdüse C, einer Mischkammer D und einem sich anschließenden Diffusor E. Die Prozessgase werden über den Einlass A und über die Treibdüse C in die Mischkammer D und dem Diffusor E eingeleitet. Die Eingabe in die Sterilisationskammer erfolgt über den Ausgang F. Durch die Treibdüse wird ein Unterdruck erzeugt, der dann aus der Sterilisationskammer über den Eingang B Prozessgase entzieht. Die Vermischung der Gase erfolgt direkt hinter der Treibdüse C in der Mischkammer D und dem Diffusor E. Durch die hohen Gasgeschwindigkeiten und dem geringeren Mischkammervolumen ist die Durchmischung deutlich höher gegenüber der direkten Eingabe in die Sterilisationskammer.

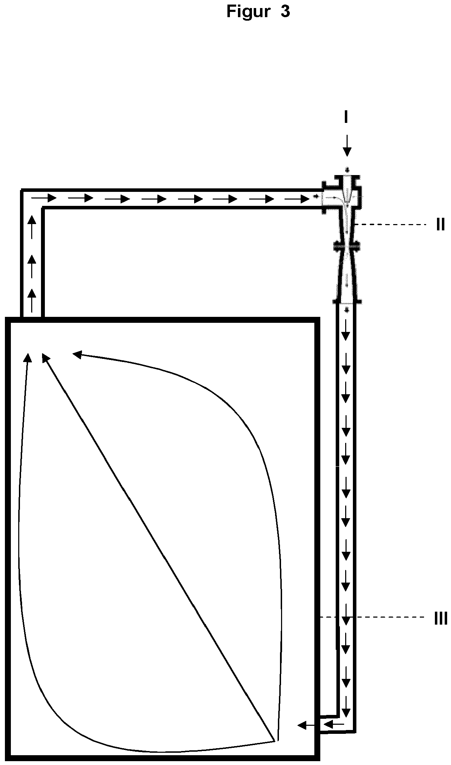

In Fig. 3 ist schematisch der Anschluss eines Mischers ohne bewegliche Teile in Form einer Strahlpumpe an eine Sterilisationskammer abgebildet. Die Gaseingabe erfolgt, wie bereits beschrieben, am Punkt I in den Mischer ohne bewegliche Teile in Form einer Strahlpumpe II. Die Zirkulation und Mischung der Prozessgase wird während der Eingabe dadurch erzeugt, dass ein unter Druck stehendes Gas (Treibstrom) in den Mischer ohne bewegliche Teile in Form einer Strahlpumpe geleitet wird. Durch Impulsaustausch wird das Gas aus der Sterilisationskammer angesaugt (Saugstrom), beschleunigt und verdichtet. Der Saugstrom mischt sich mit dem sehr schnell strömenden Treibstrom und wird dadurch beschleunigt. Die Einlassöffnung für die Gase in der Sterilisationskammer III befindet sich diagonal zu der Auslassöffnung, damit kein Bypass in der Kammer entstehen kann. Dabei werden die Gase, die vom Mischer ohne bewegliche Teile in Form einer Strahlpumpe über die Einlassöffnung in die Sterilisationskammer fließen, wieder von der vorzugsweise diagonal gegenüberliegenden oder möglichst weit entfernt liegenden Auslassöffnung angesaugt. Die Eingabe und Verteilung der Gase innerhalb der Kammer erfolgt über den Druckunterschied zwischen der Kammer und dem Eingabemedium. Der Gasfluss ist dabei derart, dass die Sterilisationsgüter im Fluß liegen bzw. für Verwirbelungen sorgen.

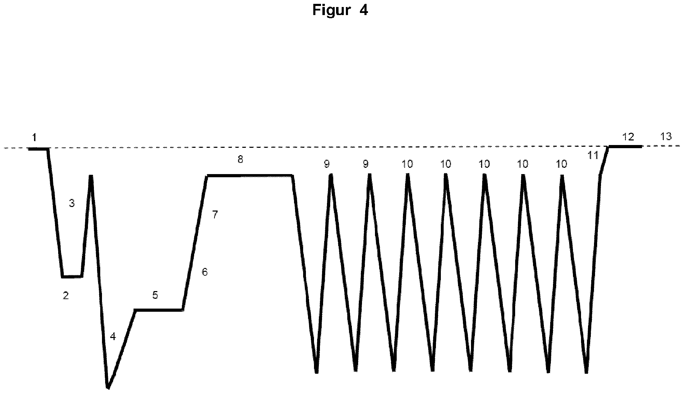

In Fig. 4 ist schematisch der Druckverlauf während der Sterilisation abgebildet. Der gesamte Prozess erfolgt im Unterdruck, wobei während des Prozesses unterschiedliche Gase eingegeben und anschließend über eine Vakuumpumpe wieder aus der Sterilisationskammer abgesaugt werden, wobei das Absaugen der Gase anhand der nach unten gerichteten Peaks verdeutlicht wird und die Plateaus (Schritt 5 und 8) zeigen, wann die Gase in der Kammer verbleiben. 1) Die Sterilisationskammer wird beladen und der Prozess gestartet. 2) Zunächst wird ein erstes Vakuum angelegt und ein Leckagetest durchgeführt, dann 3) erfolgt die Stickstoffeingabe mit anschließender Evakuierung und ein Leckagetest. 4) Es folgt die Dampfeingabe und Nachdosierung mit anschließender Dampfeinwirkzeit 5). Hieran schließt sich die Ethylenoxideingabe 6) an, gefolgt von der Stickstoffeingabe 7). Die eigentliche Sterilisation erfolgt im Schritt 8) mit der Einwirkzeit des Ethylenoxids. Durch die Spülschritte 9) mit Stickstoff und 10) mit Luft wird verbliebendes Ethylenoxid entfernt. Der gesamte Prozess endet mit dem Druckausgleich 11) und der Entladung des sterilisierten Gutes 12).

Die vorliegende Erfindung umfasst auch ein Verfahren zur Gassterilisation von Erzeugnissen in einer Sterilisationskammer mit mindestens einer Ein- und Auslassöffnung und einem Mischer ohne bewegliche Teile, dadurch gekennzeichnet, dass zumindest ein Teil der Gase während der Zugabe in die Sterilisationskammer über einen Mischer ohne bewegliche Teile zu einem homogenen Gasgemisch vermischt werden.

In einer weiteren bevorzugten Ausführungsform ist das Verfahren zur Gassterilisation von Erzeugnissen in einer Sterilisationskammer mit mindestens einer Ein- und Auslassöffnung und einem Mischer ohne bewegliche Teile, dadurch gekennzeichnet, dass zumindest ein Teil der Gase während der Zugabe in die Sterilisationskammer in einem Mischer ohne bewegliche Teile gemischt und anschließend über eine Einlassöffnung in die Sterilisationskammer geleitet wird, wobei gleichzeitig zumindest ein Teil der Gase, welche sich bereits in der Sterilisationskammer befinden, über eine Auslassöffnung aus der Sterilisationskammer dem Mischer ohne bewegliche Teile zugeleitet werden und sich dort mit einem der Gase aus der Zugabe vermischen.

Bei der Zugabe handelt es sich um das Gas, welches zum ersten Mal in diesen Kreislauf der Durchmischung eingebracht wird. Dies kann z.B. die Inertgaseingabe sein, aber auch die Zugabe von Wasserdampf oder die Zugabe des Sterilisationsgases. Mit Zugabe ist damit nicht das angesaugte Gas aus der Sterilisationskammer gemeint. Das angesaugte Gas aus der Sterilisationskammer vermischt sich mit dem Gas der Zugabe im Mischer ohne bewegliche Teile und wird wieder in die Sterilisationskammer geleitet. Dieses Gasgemisch wird dann erneut angesaugt und vermischt sich dann wieder mit dem Gas der Zugabe im Mischer ohne bewegliche Teile.

In einer bevorzugten Ausführungsform umfasst das Verfahren die Folgenden Schritte:
a) Anlegen eines Vakuums in der Sterilisationskammer,
b) Inertgaseingabe in die Sterilisationskammer,
c) Wasserdampfeingabe in die Sterilisationskammer,
d) Eingabe des Sterilisationsgases in die Sterilisationskammer,
e) Inertgaseingabe in die Sterilisationskammer,
f) Einwirkzeit für das Sterilisationsgas,
g) mehrmaliges Spülen mit Inertgas und/oder Luft,
h) Druckausgleich;
wobei das Verfahren weiterhin dadurch gekennzeichnet ist, dass zumindest bei einer der Eingaben b) - e) und/oder g) das Gas vor Eingabe in die Sterilisationskammer in einem Mischer ohne bewegliche Teile gemischt und anschließend über eine Einlassöffnung in die Sterilisationskammer geleitet wird, wobei gleichzeitig zumindest ein Teil der Gase, welcher sich bereits in der Sterilisationskammer befindet, über eine Auslassöffnung aus der Sterilisationskammer dem Mischer ohne bewegliche Teile zugeleitet wird und sich dort mit einem der Gase aus den Eingaben b) - e) und/oder g) vermischt.

Als Inertgase bezeichnet man Gase, die sehr reaktionsträge (inert) sind, sich also an nur wenigen und vorzugsweise keinen chemischen Reaktionen beteiligen, welche bei der Sterilisation ablaufen könnten. Ob man ein bestimmtes Gas für eine bestimmte Anwendung als Inertgas bezeichnet, ist allerdings dennoch vom konkreten Fall abhängig. Zu den Inertgasen gehören zum Beispiel Stickstoff und sämtliche Edelgase (Helium, Neon, Argon, Krypton, Xenon, Radon).

Prinzipiell kann das Verfahren mit jeglicher Art von Sterilisationsgasen durchgeführt werden. Hierunter fallen z.B. Ethylenoxid oder anderen Alkylenoxiden, Formaldehyd, Peressigsäure, Wasserstoffperoxid oder anderen Peroxiden und/oder Wasserdampf.

Erfindungsgemäß kann das Verfahren auch für solche Gassterilisationsverfahren angewendet werden, bei denen eine Inertisierung nicht notwendig ist. In solch einem Fall umfasst das Verfahren die folgenden Schritte:
a) Anlegen eines Vakuums in der Sterilisationskammer,
b) Wasserdampfeingabe in die Sterilisationskammer,
c) Eingabe des Sterilisationsgases in die Sterilisationskammer,
d) Einwirkzeit für das Sterilisationsgas,
e) mehrmaliges Spülen mit Luft,
f) Druckausgleich;
wobei das Verfahren weiterhin dadurch gekennzeichnet ist, dass zumindest bei einer der Eingaben b) und/oder c) und/oder e) das Gas vor Eingabe in die Sterilisationskammer in einem Mischer ohne bewegliche Teile gemischt und anschließend über eine Einlassöffnung in die Sterilisationskammer geleitet wird, wobei gleichzeitig zumindest ein Teil der Gase, welcher sich bereits in der Sterilisationskammer befindet, über eine Auslassöffnung aus der Sterilisationskammer dem Mischer ohne bewegliche Teile zugeleitet wird und sich dort mit einem der Gase aus den Eingaben b) und/oder c) und/oder e) vermischt.

### Beispiele

### Beispiel 1: Vergleich Gassterilisationsvorrichtung mit und ohne einen Mischer ohne bewegliche Teile in Form einer Strahlpumpe

Die Auswirkungen einer Strahlpumpe auf die Temperatur- und somit auch Gasverteilung während der Sterilisation mit Ethylenoxid wurden ermittelt und mit einer Vorrichtung ohne eine Strahlpumpe verglichen.

Die Ethylenoxid Sterilisation ist eine chemische Sterilisation, die temperaturabhängig ist. Eine Erhöhung der Prozesstemperatur um 10°C ergibt eine Verdoppelung der Reaktionsgeschwindigkeit bzw. eine Halbierung der Einwirkzeit. Daher ist die Temperatur ein wichtiger Prozessparameter für die Sterilisation. Um Unterschiede in der Effektivität und Homogenität des Sterilisationsprozesses zu dokumentieren, wurde die Aufnahme der Temperaturverteilung während der Ethylenoxid Einwirkzeit überprüft. Hierfür wurden Temperatursensoren in dem Sterilgut (Paletten mit medizinischen Einmalartikeln) platziert und die Werte aufgezeichnet. Die Aufnahme der Temperaturverteilung wurde dreimal wiederholt, um die Reproduzierbarkeit der Ergebnisse zu gewährleisten. Da die Temperaturzuführung über die Zugabe von Wasserdampf erfolgt, kann dadurch die Temperaturverteilung auch als Indiz für die Gasverteilung innerhalb der Sterilisationskammer und des Sterilgutes verwendet werden.

### Anlage ohne Strahlpumpe:

Eingesetzt wurden 43 Temperaturfühler Typ Data Trace, die in den Paletten verteilt wurden. Jede Minute wurde ein Messpunkt aufgenommen. Zu jeder Messreihe wurde der Maximumwert und Minimumwert aller 43 Sensoren ermittelt.

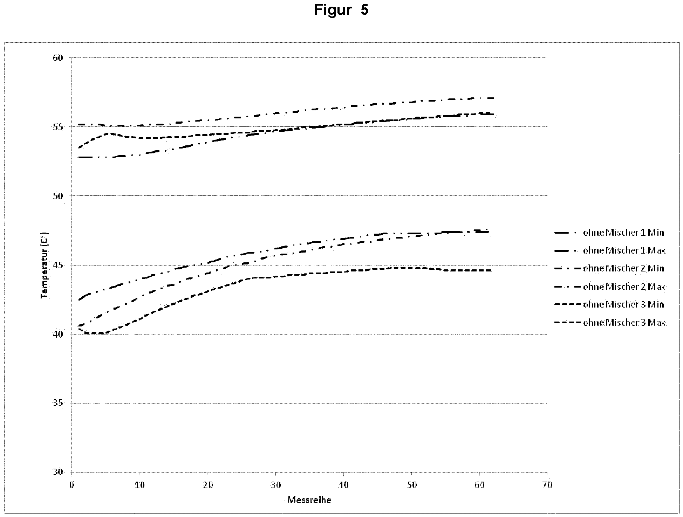

Die Ergebnisse der Messungen in der Anlage ohne eine Strahlpumpe zeigt die Fig. 5. Die Fig. 5 zeigt die Min/Max Werte der drei Validierungläufe. Aus allen Messreihen während der Einwirkzeit wurden die maximalen Temperaturunterschiede ermittelt. Das Ergebnis der maximalen Temperaturunterschiede ist in der Tabelle 1 abgebildet. Im Mittel aus allen drei Läufen ergibt sich eine Temperaturabweichung von 12,6 °C [(10,3 + 14,6 + 13,1)/3] zwischen der Maximumtemperatur und Minimumtemperatur.

**Tab.1**

| **Validierungslauf 1 Anlage ohne Mischer** | | |
|---|---|---|
| Min (°C) | Max (°C) | Abweichung |
| 42,50 | 52,80 | 10,30 |
| | | |

| **Validierungslauf 2 Anlage ohne Mischer** | | |
|---|---|---|
| Min (°C) | Max (°C) | Abweichung |
| 40,60 | 55,20 | 14,60 |
| | | |

| **Validierungslauf 3 Anlage ohne Mischer** | | |
|---|---|---|
| Min (°C) | Max (°C) | Abweichung |
| 40,40 | 53,50 | 13,10 |

### Anlage mit Strahlpumpe:

Unter vergleichbaren Bedingungen wurde derselbe Prozess mit einer Anlage durchgeführt, in der eine Strahlpumpe verbaut wurde.

Eingesetzt wurden 45 Temperaturfühler Typ Data Trace, die in den Paletten verteilt wurden. Jede Minute wurde ein Messpunkt aufgenommen. Zu jeder Messreihe wurde der Maximumwert und Minimumwert aller 45 Sensoren ermittelt.

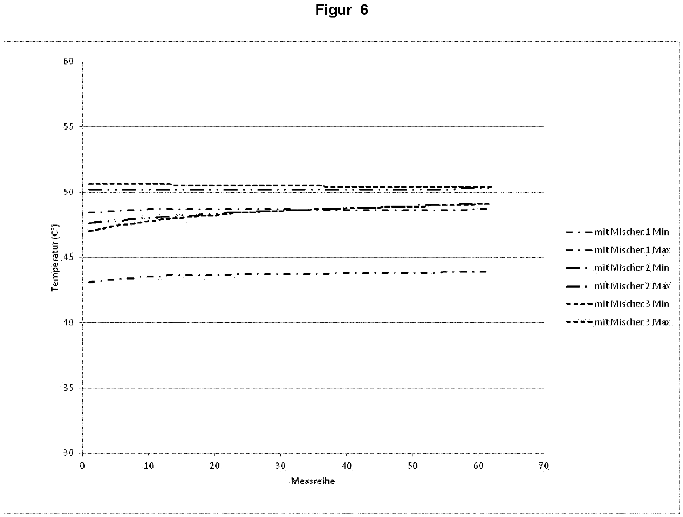

Die Ergebnisse der Messungen in der Anlage ohne eine Strahlpumpe zeigt die Fig. 6. Die Fig. 6 zeigt die Min/Max Werte der drei Validierungläufe. Aus allen Messreihen wurden während der Einwirkzeit die maximalen Temperaturunterschiede ermittelt. Das Ergebnis der maximalen Temperaturunterschiede ist in der Tabelle 2 abgebildet. Im Mittel aus allen drei Läufen ergibt sich eine Temperaturabweichung von 3,8 °C [(5,3 + 2,6 + 3,6)/3] zwischen der Maximum- und Minimumtemperatur.

**Tab.2**

| **Validierungslauf 1 Anlage mit Strahlpumpe** | | |
|---|---|---|
| Min (°C) | Max (°C) | Abweichung |
| 43,10 | 48,40 | 5,30 |
| | | |

| **Validierungslauf 2 Anlage mit Strahlpumpe** | | |
|---|---|---|
| Min (°C) | Max (°C) | Abweichung |
| 47,60 | 50,20 | 2,60 |
| | | |

| **Validierungslauf 3 Anlage mit Strahlpumpe** | | |
|---|---|---|
| Min (°C) | Max (°C) | Abweichung |
| 47,00 | 50,60 | 3,60 |

### Verqleichbarkeit der Ergebnisse:

Sensoren: In beiden Validierungsstudien (mit und ohne Strahlpumpe) wurden dieselben Sensoren eigesetzt.

Prozessparameter: Wie bereits oben ausgeführt wurde dem Produkt die Temperatur über die Dampfeigabe zugeführt. Bei beiden Anlagen erfolgte die Dampfzugabe über zwei Eingaben. Nach der Eingabe erfolgte die Dampfeinwirkzeit.

Die Prozessparameter waren dabei Folgendermaßen:

**Tab.3**

| **Prozessparameter** | | **Ohne Strahlpumpe** | **Mit Strahlpumpe** |
|---|---|---|---|
| 1. | Eingabe | | |
| | Start: | 50 mbar | 50 mbar |
| | Ende: | 125 mbar | 125 mbar |
| | Delta | 75 mbar | 75 mbar |
| 2. | Eingabe | | |
| | Start: | 75 mbar | 80 mbar |
| | Ende: | 125 mbar | 125 mbar |
| | Delta | 50 mbar | 45 mbar |
| Einwirkzeit | | 30 Minuten bei 120-125 mbar | 30 Minuten bei 120-125 mbar |

Der Unterschied von 5 mbar bei der zweiten Dampfeingabe kann im Gesamtprozess vernachlässigt werden, bzw. müsste sich sogar negativ auf die Temperaturverteilung in der Anlage mit der Strahlpumpe auswirken, was nicht der Fall war. Somit sind die Prozessparameter der beiden Anlagen vergleichbar.

Beladung. Alle Validierungsläufe wurden mit medizinischen Plastikeinmalartikeln (Spritzen und IV-Sets/Infusionsübergangsleitungen) durchgeführt.
- Beladung Anlage ohne Strahlpumpe: 5 ml Spritze (Dichte 116 kg/m3, Material PP, PE)
- Beladung Anlage mit Strahlpumpe: IV-Sets (Dichte 123-140 kg/m³, Material PVC, PS)

Die physikalischen Eigenschaften der Kammerbeladung sind hinsichtlich der Temperaturaufnahme vergleichbar. Der Material und Dichteunterschied kann vernachlässigt werden.

Sterilisationskammer: Beide Sterilisationskammern sind vom gleichen Lieferanten und die Verteilung der Heizrippen ist identisch.

Die Sterilisationskammern unterscheiden sich in Ihrer Abmessung und dem Design der Gaseintrittsöffnungen.

**Tab.4**

| | **Ohne Strahlpumpe** | **Mit Strahlpumpe** |
|---|---|---|
| Volumen | 49,5 m³ | 55,1 m³ |
| Produktvolumen | 30,6 m³ | 34 m³ |
| Dampfeinlässe und Anordnungen | 9 Einlassöffnungen auf beiden Längsseiten der Kammer verteilt | 5 Einlassöffnungen auf einer Seite und 6 Auslassöffnungen gegenüberliegend |

Bedingt durch die unterschiedlichen Anordnungen, die durch den Einbau der Strahlpumpe begründet ist, kann ein Effekt nicht ausgeschlossen werden. Daher wurde eine zweite Versuchsreihe durchgeführt, in dem eine Anlage verbaut wurde, in der die Strahlpumpe durch einen Schieber oder ein Ventil vom Rest der Anlage getrennt werden kann.

### Beispiel 2: Vergleich Gassterilisationsvorrichtung mit Strahlpumpe trennbar durch einen Schieber vom Rest der Anlage

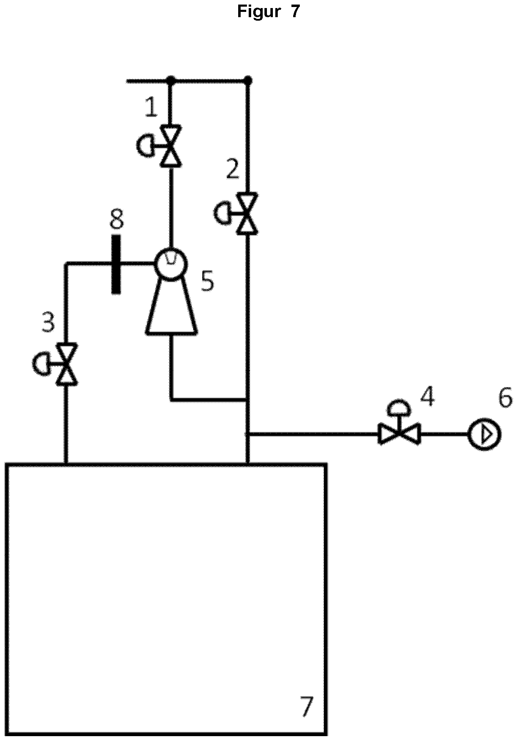

Es wurde ein Validierungslauf analog im Beispiel 1 durchgeführt. Um die Wirkung der Strahlpumpe auszuschließen wurde ein verschließbarer Schieber zwischen den Zugang an der Strahlpumpe 5 und dem Ventil 3 verbaut (vgl. Fig.7). Dadurch konnte die Wirkung der Strahlpumpe unterbunden werden, da keine Prozessgase aus der Kammer angesaugt und in der Mischkammer gemischt werden können.

### Vergleich der Temperaturabweichungen:

Die Messergebnisse der Temperatursensoren wurden mit dem Validierungslauf aus Beispiel 1 (Anlage mit der Strahlpumpe) verglichen. Hierfür wurden die jeweiligen Mittelwerte aller Temperatursensoren zu einem Prozesszeitpunkt ermittelt und zwischen den beiden Versuchsläufen verglichen.

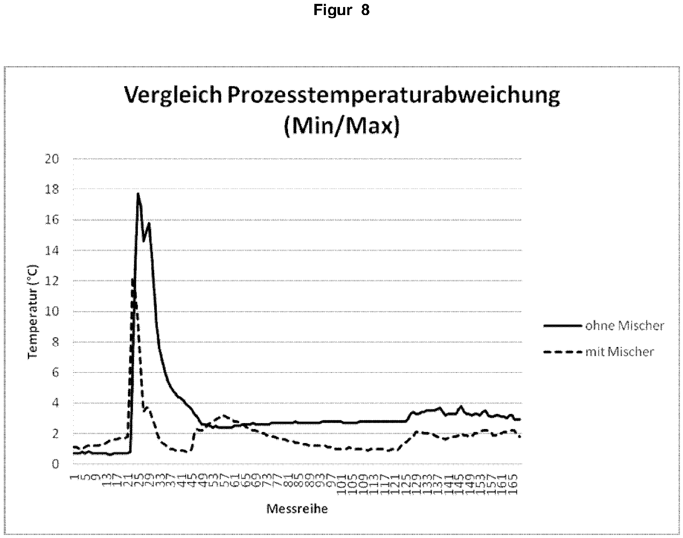

Die beiden Temperaturverläufe (mittlere Prozesstemperaturabweichung aller Sensoren) sind in der Fig. 8 abgebildet. Wie sich aus der Fig. 8 entnehmen lässt, wird durch den Einsatz des Mischers eine homogenere Verteilung der Temperatur innerhalb der Beladung erreicht. Die maximale Abweichung verringert sich um 50 % von 18°C auf 12°C (vgl. Fig. 8).

### Vergleich der Temperaturmittelwerte:

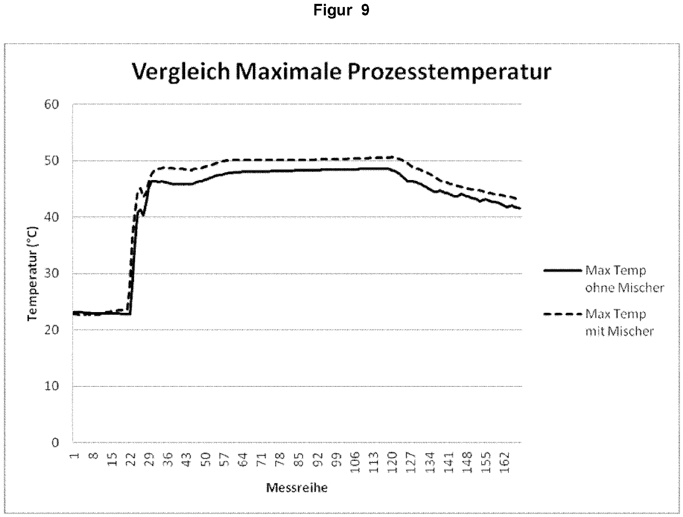

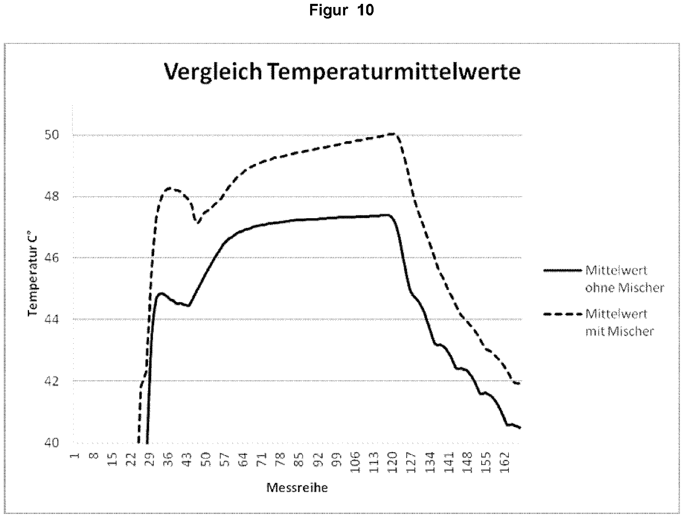

Desweiteren wurde die mittlere Prozesstemperatur aller Temperatursensoren zu allen Zeitpunkten im Prozess ermittelt und miteinander verglichen. Das Ergebnis dieses Vergleichs ist in der Fig. 9 abgebildet. Es hat sich gezeigt, dass die mittlere Prozesstemperatur (Mittelwert aller Sensoren zu einem Zeitpunkt) durch den Einsatz der Strahlpumpe bei ansonsten gleichen Prozessbedingungen deutlich ansteigt. In der Fig. 10 ist noch einmal ein Vergleich der Temperaturmittelwerte für den Bereich der besonders wichtigen Dampf und Ethlyenoxid Einwirkzeit abgebildet. Gerade hier zeigt sich eine deutlich Steigerung der mittleren Temperatur in der Anlage mit der Strahlpumpe.

Somit ist festzuhalten, dass die Verbesserung der Temperaturverteilung in der Sterilisationskammer eindeutig auf den Einsatz der Strahlpumpe zurückzuführen ist. Durch die Mischung der Prozessgase vor der eigentlichen Eingabe in die Sterilisationskammer ergibt sich ein homogeneres Gasgemisch, was durch die gleichmäßigere Temperaturverteilung erkennbar ist.

### Referenzzeichenliste

Bezugszeichenliste Figur 1
   - 1: Einlassventil Mischer ohne bewegliche Teile
   - 2: Einlassventil ohne Mischer ohne bewegliche Teile
   - 3: Mischventil Kammer für Mischer ohne bewegliche Teile
   - 4: Auslassventil
   - 5: Mischer ohne bewegliche Teile
   - 6: Vakuumpumpe
   - 7: Sterilisationskammer
Bezugszeichenliste Figur 2
   - A: Einlass Prozessgase
   - B: Prozessgase aus der Sterilisationskammer
   - C: Treibdüse
   - D: Mischkammer
   - E: Diffusor
   - F: Eingabe in die Sterilisationskammer
Bezugszeichenliste Figur 3
   - I: Gaseingabe
   - II: Mischer ohne bewegliche Teile in Form einer Strahlpumpe
   - III: Sterilisationskammer
Bezugszeichenliste Figur 4
   - 1): Beladen und Prozessstart
   - 2): Erstes Vakuum und Leckagetest
   - 3): Stickstoffeingabe und Leckagetest
   - 4): Dampfeingabe und Nachdosierung
   - 5): Dampfeinwirkzeit
   - 6): Ethylenoxideingabe
   - 7): Stickstoffeingabe
   - 8): Ethylenoxid Einwirkzeit
   - 9): Stickstoffspülung
   - 10): Luftspülung
   - 11): Druckausgleich
   - 12): Entladung und Prozessende
   - 13): Normaldruck
Bezugszeichenliste Figur 7
   - 1: Einlassventil Mischer ohne bewegliche Teile
   - 2: Einlassventil ohne Mischer ohne bewegliche Teile
   - 3: Mischventil Kammer für Mischer ohne bewegliche Teile
   - 4: Auslassventil
   - 5: Mischer ohne bewegliche Teile
   - 6: Vakuumpumpe
   - 7: Sterilisationskammer
   - 8: Einbaupunkt des Schiebers

### Figurenbeschreibung

**Figur 1** Fließschema mit einem Mischer ohne bewegliche Teile im Rohrleitungssystem einer Gassterilisationsvorrichtung.
**Figur 2** Schematische Ansicht: Mischer ohne bewegliche Teile in Form einer Strahlpumpe.
**Figur 3** Schematische Darstellung: Anschluss eines Mischers ohne bewegliche Teile in Form einer Strahlpumpe an eine Sterilisationskammer.
**Figur 4** Schematische Darstellung: Druckkurve während des Sterilisationsprozesses.
**Figur 5** Temperatur Min/Max Werte der drei Validierungsläufe in der Anlage ohne einen Mischer.
**Figur 6** Temperatur Min/Max Werte der drei Validierungsläufe in der Anlage mit einem Mischer.
**Figur 7** Fließschema mit einem Mischer ohne bewegliche Teile im Rohrleitungssystem einer Gassterilisationsvorrichtung, welcher vom Rest der Anlage getrennt werden kann.
**Figur 8** Temperaturverläufe (mittlere Prozesstemperaturabweichung aller Sensoren) in der Anlage mit Mischer ohne bewegliche Teile und der Anlage mit abgeriegeltem Mischer ohne bewegliche Teile.
**Figur 9** Temperaturverläufe (mittlere Prozesstemperatur aller Sensoren) in der Anlage mit Mischer ohne bewegliche Teile und der Anlage mit abgeriegeltem Mischer ohne bewegliche Teile.
**Figur 10** Temperaturverläufe (mittlere Prozesstemperatur aller Sensoren) in der Anlage mit Mischer ohne bewegliche Teile und der Anlage mit abgeriegeltem Mischer ohne bewegliche Teile im Bereich der Dampf und Ethylenoxid Einwirkzeit.

## Patentansprüche

1. Vorrichtung zur Gassterilisation von Erzeugnissen, umfassend eine Sterilisationskammer und einen Mischer ohne bewegliche Teile.

2. Vorrichtung gemäß Anspruch 1, wobei die Sterilisationskammer mindestens eine Einlassöffnung und mindestens eine Auslassöffnung für Gase aufweist.

3. Vorrichtung gemäß Anspruch 1 - 2, wobei der Mischer ohne bewegliche Teile einen Einlass, für ein oder mehrere Prozessgase, einen Auslass, zur Eingabe der Gase in die Sterilisationskammer und einen Einlass für Prozessgase, die aus der Sterilisationskammer kommen, aufweist.

4. Vorrichtung gemäß Anspruch 1 - 3, **dadurch gekennzeichnet, dass** der Mischer ohne bewegliche Teile in Form einer Strahlpumpe ausgebildet ist, welche eine Treibdüse, eine Mischkammer und einen Diffusor umfasst.

5. Vorrichtung gemäß Anspruch 1 - 2, **dadurch gekennzeichnet, dass** der Mischer ohne bewegliche Teile in Form eines statischen Mischers ausgebildet ist.

6. Vorrichtung zur Gassterilisation gemäß Anspruch 1, umfassend einen Mischer ohne bewegliche Teile (5), Ventile (1, 3) und eine Sterilisationskammer (7), wobei die Sterilisationskammer (7) mindestens eine Einlassöffnung und mindestens eine Auslassöffnung aufweist und der Mischer ohne bewegliche Teile derart angeordnet ist, dass er über eine erste Leitung, die in einer ersten Öffnung im Mischer ohne bewegliche Teile mündet, mit der mindestens einen Auslassöffnung der Sterilisationskammer (7) verbunden ist und mit einer zweiten Leitung, die in einer zweiten Öffnung im Mischer ohne bewegliche Teile mündet, mit der mindestens einen Einlassöffnung der Sterilisationskammer (7) verbunden ist und eine dritte gaszuführende Leitung in eine dritte Öffnung im Mischer ohne bewegliche Teile mündet, wobei das Ventil (3) in der ersten Leitung zwischen der Auslassöffnung der Sterilisationskammer (7) und dem Mischer ohne bewegliche Teile (5) und das Ventil (1) in der dritten gaszuführenden Leitung zum Mischer ohne bewegliche Teile (5) angeordnet ist.

7. Vorrichtung gemäß Anspruch 4 - 5, zusätzlich umfassend eine gaszuführende Bypass-Leitung mit einem Ventil (2), wobei die gaszuführende Bypass-Leitung derart angeordnet ist, dass sie den Mischer ohne bewegliche Teile überbrückt und direkt in der Sterilisationskammer (7) mündet.

8. Verfahren zur Gassterilisation von Erzeugnissen in einer Sterilisationskammer umfassend mindestens eine Ein- und Auslassöffnung und einen Mischer ohne bewegliche Teile, **dadurch gekennzeichnet, dass** zumindest ein Teil der Gase während der Zugabe in die Sterilisationskammer über einen Mischer ohne bewegliche Teile zu einem homogenen Gasgemisch vermischt werden.

9. Verfahren gemäß Anspruch 8, wobei zumindest ein Teil der Gase während der Zugabe in die Sterilisationskammer in einem Mischer ohne bewegliche Teile gemischt und anschließend über eine Einlassöffnung in die Sterilisationskammer geleitet wird, wobei gleichzeitig zumindest ein Teil der Gase, welche sich bereits in der Sterilisationskammer befinden, über eine Auslassöffnung aus der Sterilisationskammer dem Mischer ohne bewegliche Teile zugeleitet wird und sich dort mit einem der Gase aus der Zugabe vermischt.

10. Verfahren gemäß Anspruch 8 oder 9, umfassend die folgenden Schritte:
a) Anlegen eines Vakuums in der Sterilisationskammer,
b) Inertgaseingabe in die Sterilisationskammer,
c) Wasserdampfeingabe in die Sterilisationskammer,
d) Eingabe des Sterilisationsgas in die Sterilisationskammer,
e) Inertgaseingabe in die Sterilisationskammer,
f) Einwirkzeit für das Sterilisationsgas,
g) mehrmaliges Spülen mit Inertgas und/oder Luft,
h) Druckausgleich;
wobei das Verfahren weiterhin **dadurch gekennzeichnet ist, dass** zumindest bei einer der Eingaben b) - e) und/oder g) das Gas vor Eingabe in die Sterilisationskammer in einem Mischer ohne bewegliche Teile gemischt und anschließend über mindestens eine Einlassöffnung in die Sterilisationskammer geleitet wird, wobei gleichzeitig zumindest ein Teil der Gase, welche sich bereits in der Sterilisationskammer befinden, über mindestens eine Auslassöffnung aus der Sterilisationskammer dem Mischer ohne bewegliche Teile zugeleitet werden und sich dort mit einem der Gase aus den Eingaben b) - e) und/oder g) vermischt.

11. Verfahren gemäß Anspruch 8 oder 9, umfassend die folgenden Schritte:
a) Anlegen eines Vakuums in der Sterilisationskammer,
b) Wasserdampfeingabe in die Sterilisationskammer,
c) Eingabe des Sterilisationsgases in die Sterilisationskammer,
d) Einwirkzeit für das Sterilisationsgas,
e) mehrmaliges Spülen mit Luft,
f) Druckausgleich.
wobei das Verfahren weiterhin **dadurch gekennzeichnet ist, dass** zumindest bei einer der Eingaben b) und/oder c) und/oder e) das Gas vor Eingabe in die Sterilisationskammer in einem Mischer ohne bewegliche Teile gemischt und anschließend über eine Einlassöffnung in die Sterilisationskammer geleitet wird, wobei gleichzeitig zumindest ein Teil der Gase, welche sich bereits in der Sterilisationskammer befinden, über eine Auslassöffnung aus der Sterilisationskammer dem Mischer ohne bewegliche Teile zugeleitet wird und sich dort mit einem der Gase aus den Eingaben b) und/oder c) und/oder e) verm ischt.

12. Verfahren gemäß einem der Ansprüche 8 - 11, wobei das Sterilisationsgas ausgewählt ist aus der Gruppe bestehend aus Ethylenoxid, Formaldehyd, Peressigsäure, Wasserstoffperoxid und/oder Wasserdampf.
